# FASCICULE DE BREVET EUROPEEN

(11) **EP 0 457 643 B1**
(45) Date de publication et mention de la délivrance du brevet: **12.10.1994**
(21) Numéro de dépôt: 91401163.0
(22) Date de dépôt: 02.05.1991
(51) Int. Cl.: C07C 2/76, B01J 19/24

(54) **Procédé de conversion thermique du méthane et réacteur pour la mise en oeuvre du procédé**
Verfahren zur thermischen Umsetzung von Methan und Vorrichtung zur Durchführung des Verfahrens
Process for the thermal conversion of methane and reactor to be used therefor

(30) Priorité: 17.05.1990 FR 9006294
(43) Date de publication de la demande: 21.11.1991
(73) Titulaire: INSTITUT FRANCAIS DU PETROLE, 92502 Rueil-Malmaison (FR)
(72) Inventeur: Alagy, Jacques, F-69260 Charbonnieres (FR); Broutin, Paul, F-69130 Ecully (FR); Busson, Christian, F-69260 Charbonniere (FR); Weill, Jérôme, F-69005 Lyon (FR)

(56) Documents cités:
- EP-A- 0 323 287

## Description

L'invention concerne un procédé de conversion thermique du méthane en hydrocarbures de poids moléculaires plus élevés et le dispositif pour la mise en oeuvre de ce procédé. Elle concerne plus particulièrement un procédé de conversion ou craquage thermique du méthane dans un réacteur comportant des moyens de chauffage électrique et permettant, par couplage thermique déshydrogénant de cette molécule, la production d'acétylène, d'éthylène, de benzène et d'un peu de coke.

Toutes les sources de méthane bien connues de l'homme du métier peuvent être utilisées. Comme source de méthane très courante, on peut citer le gaz naturel. Une liste non exhaustive de ces sources a par exemple été fournie dans la demande de brevet européen au nom de la demanderesse EP-A-323287. Dans la majorité des cas, le gaz contenant du méthane que l'on introduit dans le réacteur contient de 1 à 90% d'au moins un autre gaz et parfois même plus.

Dans la demande de brevet européen EP-A-323287, la demanderesse a décrit un procédé de conversion thermique du méthane en hydrocarbures de poids moléculaires plus élevés comportant des moyens de chauffage électrique avec tranfert de chaleur au mélange gazeux, contenant le méthane à convertir, à travers les parois étanches de gaines en matière céramique qui isolent lesdits moyens de chauffage du mélange gazeux contenant le méthane. Dans ce procédé, la zone de chauffage est chauffée par apport d'énergie électrique à l'aide de résistances électriques et la chaleur dégagée par effet Joule dans ces résistances est transmise, principalement par radiation, aux gaines en matériau céramique disposées autour des résistances de façon non jointive. Les charges gazeuses, qui circulent sensiblement perpendiculairement à l'axe des gaines chauffées, sont chauffées essentiellement par convection et par radiation. Dans la réalisation de ce procédé, on définit au sein du réacteur deux espaces :
- d'une part, l'espace de réaction ou espace process, à l'extérieur des gaines protégeant les résistances, dans lequel circule le mélange de gaz contenant du méthane,
- d'autre part, l'espace des résistances formé par le volume compris entre les résistances proprement dites et les gaines d'isolement, dans lequel on introduit de préférence un gaz inerte, c'est-à-dire un gaz exempt de méthane ou de tout hydrocarbure susceptible d'une réaction de conversion thermique ou de tout composé susceptible de réagir violemment avec le méthane ou l'hydrogène. Ce gaz est également choisi de manière à ce qu'il n'endommage pas les résistances utilisées et ne provoque pas un vieillissement accéléré de ces résistances.

L'un des problèmes les plus importants lorsque l'on réalise la conversion thermique du méthane est lié à la formation du coke. En effet, lorqu'il se forme en quantité trop importante, celui-ci risque d'endommager le four avant les opérations de décokage et par ailleurs, sur le plan économique, sa formation représente une perte importante, aussi bien en ce qui concerne l'énergie électrique consommée que le méthane consommé pour sa formation. Ce problème, bien connu de l'homme du métier, est partiellement résolu par l'introduction dans le mélange de gaz, contenant le méthane à convertir, d'une quantité d'hydrogène représentant de 1 à 90% en volume par rapport au volume total de gaz. Malgré cette précaution, la formation de coke, principalement au niveau des parois des gaines et sur les autres surfaces de température élevée en contact avec le mélange de gaz contenant le méthane, n'est pas complètement éradiquée.

Ceci explique que dans la mise en oeuvre de ce procédé de conversion du méthane dans un four de pyrolyse chauffé électriquement, il soit souhaitable :
- d'avoir dans la zone process une quantité d'hydrogène relativement importante,
- de disposer de résistances électriques capable de délivrer à haute température une grande quantité d'énergie par unité de surface et par unité de temps,
- d'avoir des conditions permettant de bons transferts thermiques de manière à ce que la température des éléments chauffant, ainsi que celle des surfaces des gaines en contact avec le mélange de gaz contenant du méthane, ne soit pas trop supérieure à la température souhaitée pour effectuer la conversion du méthane.

Dans la mise en oeuvre de ce procédé, il a été précisé qu'il est préférable que l'espace des résistances soit rempli par un fluide gazeux tel que l'azote, le gaz carbonique ou l'air. L'utilisation de l'air ne peut se concevoir que si l'étanchéité assurée par les gaines entre l'espace process et l'espace des résistances est parfaite. En effet, il y aurait autrement un risque important de former un mélange gazeux, à très haute température, comprenant de l'oxygène, du méthane et de l'hydrogène et donc un risque d'explosion. La réalisation d'un système parfaitement étanche est extrêmement difficile et de plus nécessite l'utilisation de céramique de très grande étanchéité et donc de très grande qualité, c'est-à-dire une céramique dont la densité est proche de la densité théorique, exempte de porosité ouverte.

L'emploi d'une telle céramique est d'un coût très élevé, ce qui pénalise fortement le procédé. On est donc de fait conduit à accepter l'utilisation de gaines dont l'étanchéité n'est pas parfaite et à utiliser, soit de l'azote avec le risque, non négligeable vu la température de peau des résistances, de formation, dans le cas de résistances en carbure de silicium, de nitrure de silicium, ce qui est en principe sans conséquences sur la tenue mécanique des résistances, mais provoque une variation de la résistivité de ces éléments chauffants et donc accélère leur vieillissement, et ce d'autant plus que la température de l'élément est plus élevée et que l'énergie fournie par l'élément est plus importante, soit du dioxyde de carbone ou gaz carbonique ce qui, même si le débit de fuite depuis l'espace des résistances vers l'espace process est faible, apporte inévitablement des perturbations au niveau des séparations des produits formés au cours de la conversion thermique du méthane en les compliquant, d'une part, par sa présence et, d'autre part, par la présence de monoxyde de carbone et d'eau qui se forme inévitablement par réaction entre le dioxyde de carbone, le méthane, le coke et l'hydrogène dans l'espace process.

Un des objets de l'invention est de remédier aux inconvénients décrits ci-avant. Les objectifs que l'on se propose d'atteindre et qui répondent aux problèmes soulevés par l'art antérieur sont essentiellement les suivants :
- limiter au maximum la formation de coke, en particulier sur les surfaces chaudes telles que par exemple les parois des gaines entourant les résistances,
- utiliser comme gaz dans l'espace des résistances un gaz ou un mélange de gaz comprenant un gaz déja présent dans le mélange de gaz circulant dans l'espace process, ce qui permet d'employer des gaines dont l'étanchéité n'a pas besoin d'être très grande,
- améliorer les échanges thermiques entre le mélange gazeux contenant du méthane et les surfaces chaudes en contact avec ce mélange,
- augmenter la fiabilité du dispositif.

La présente invention propose un procédé et un dispositif pour sa mise en oeuvre apportant des améliorations notables par rapport aux réalisations selon l'art antérieur telles que par exemple une mise en oeuvre plus facile, plus souple et mieux contrôlée et un coût moins élevé, aussi bien au niveau des investissements qu'au niveau des utilités.

Plus particulièrement, l'invention concerne un procédé de conversion thermique du méthane en hydrocarbures de poids moléculaires plus élévés dans une zone de réaction, de forme allongée selon une direction (un axe), comprenant une zone de chauffage et une zone de refroidissement, faisant suite à ladite zone de chauffage, dans lequel on fait circuler, dans la zone de chauffage, un mélange gazeux, renfermant du méthane, selon une direction d'écoulement sensiblement parallèle à la direction (à l'axe) de la zone de réaction, ladite zone de chauffage comportant une pluralité de moyens de chauffage électrique disposés en nappes, sensiblement parallèles entre elles, formant en projection transversale un faisceau à pas triangulaire, carré ou rectangulaire, lesdits moyens de chauffage étant regroupés par sections successives tranversales sensiblement perpendiculaires à la direction ( à l'axe) de la zone de réaction, indépendantes entre elles et alimentées en énergie électrique de façon à déterminer au moins deux parties dans la zone de chauffage, la première partie permettant de porter la charge jusqu'à une température au plus égale à environ 1500°C, et la deuxième partie, subséquente de la première partie, permettant de maintenir la charge a une température sensiblement égale a la température maximum à laquelle elle a été portée dans ladite première partie, et dans lequel on refroidit les effluents de la zone de chauffage par introduction dans la zone de refroidissement d'un fluide de refroidissement, puis on recueille lesdits hydrocarbures de poids moléculaires plus élevés à l'extrémité de la zone réactionnelle, ledit procédé étant caractérisé en ce que les moyens de chauffage électrique sont isolés du contact direct avec le mélange gazeux renfermant du méthane par des gaines dans lesquelles on introduit un gaz contenant de l'hydrogène, lesdites gaines ayant une perméabilité appropriée et le gaz étant introduit à l'intérieur desdites gaines à une pression telle qu'il y a diffusion, au moins en certains points, d'au moins une partie de l'hydrogène depuis l'intérieur desdites gaines vers l'extérieur desdites gaines, cet hydrogène pouvant se diluer alors dans ledit mélange gazeux.

La zone de chauffage est chauffée par apport d'énergie électrique à travers des moyens de chauffage tels que des résistances électriques, la chaleur dégagée par effet Joule dans ces résistances est transmise principalement par radiation aux gaines disposées autour des résistances de façon non jointive. Ces gaines sont habituellement en matériau céramique ou en tout matériau réfractaire supportant les températures requises et les atmosphères réductrices et oxydantes du milieu, comme par exemple certains nouveaux alliages métalliques de la firme KANTHAL SA comme le KANTHAL AF, ou le KANTHAL APM. Le mélange gazeux, contenant du méthane, qui circule dans la zone de chauffage de manière sensiblement perpendiculaire à l'axe des gaines, est chauffé essentiellement par convection et par radiation.

Le couplage thermique déshydrogénant du méthane est une réaction fortement endothermique et nécessite l'obtention, à un niveau élevé de température, de l'ordre de 1100 à 1500°C, d'une densité de flux thermique très importante. Il est nécessaire que l'apport maximum de chaleur soit effectué dans la zone où s'effectuent les réactions endothermiques de craquage et de déshydrogénation ; par ailleurs, compte tenu de la réactivité des produits formés, comme l'acétylène ou l'éthylène, il est nécessaire d'avoir un temps de contact contrôlé, relativement court, suivi d'une trempe rapide, de façon à obtenir un profil de température de type "carré" et à éviter une trop grande formation de coke.

Les échanges thermiques sont l'un des éléments clé pour ce type de réaction très endothermique où il est nécessaire de transférer des quantités d'énergie très importantes depuis les résistances vers le mélange gazeux contenant du méthane dénommé ci-après gaz process. Au cours de l'étude préliminaire effectuée par la demanderesse sur les échanges thermiques dans un four à pyrolyse construit selon le modèle utilisé dans la présente invention, on s'est aperçu que l'échange de chaleur depuis la résistance vers la gaine est un échange essentiellement radiatif, mais par contre il n'y a pratiquement pas d'échange radiatif entre la gaine et le gaz process. En effet, celui-ci est habituellement essentiellement constitué d'un mélange hydrogèneméthane, mélange qui n'absorbe pratiquement pas ou très peu le rayonnement émis par les gaines. Le tranfert thermique, entre le gaz process et les gaines, est donc dans le cas envisagé dans la présente invention essentiellement un tranfert par convection. Dans un tel cas, la qualité des échanges thermiques sera directement liée à la surface d'échange disponible et au rapport surface/volume.

Ainsi, si la surface d'échange est relativement faible, il sera nécessaire, pour obtenir une température de gaz process donnée correpondant à un taux de conversion préalablement choisi, d'augmenter la température des gaines, dans des proportions d'autant plus importantes que cette surface est faible, ce qui implique un risque accru de formation de coke et également la nécessité d'augmenter la température des résistances, ce qui entraîne un vieillisssement plus rapide de ces résistances ou même, si le taux de conversion préalablement choisi est très élevé, la quantité d'énergie à tranférer devient très grande et le risque de détérioration des résistances augmente très fortement.

Les parois participent de manière importante à l'échange thermique, puisqu'elles sont capables d'absorber le rayonnement émis par les gaines et par conséquent les températures des gaines et des parois ont tendance à s'équilibrer. Il est alors possible d'augmenter notablement la surface d'échange et pratiquement de la doubler en modifiant la conception du dispositif de la manière suivante :
alors que, dans la conception initiale, les gaines protégeant les résistances et permettant le tranfert de chaleur au gaz process étaient de préférence disposées en quinconce, selon la présente invention elles seront de préférence alignées, ce qui permet de constituer n rangées ou nappes de m résistances dans le sens de la longueur (pour un nombre total de résistances égal à nxm), et chaque nappe est séparée de la suivante par une paroi en matériau réfractaire.

Par radiation, la température de ces parois augmente et a tendance à atteindre la même valeur que celle des gaines entourant les résistances. Ces parois participeront donc également au chauffage par convection du gaz process. Ainsi, dans cette forme de réalisation, la surface d'échange étant pratiquement doublée, on pourra obtenir la même température de gaz process avec une température des gaines et des parois relativement plus faible, ce qui permet en conséquence une diminution de la formation de coke.

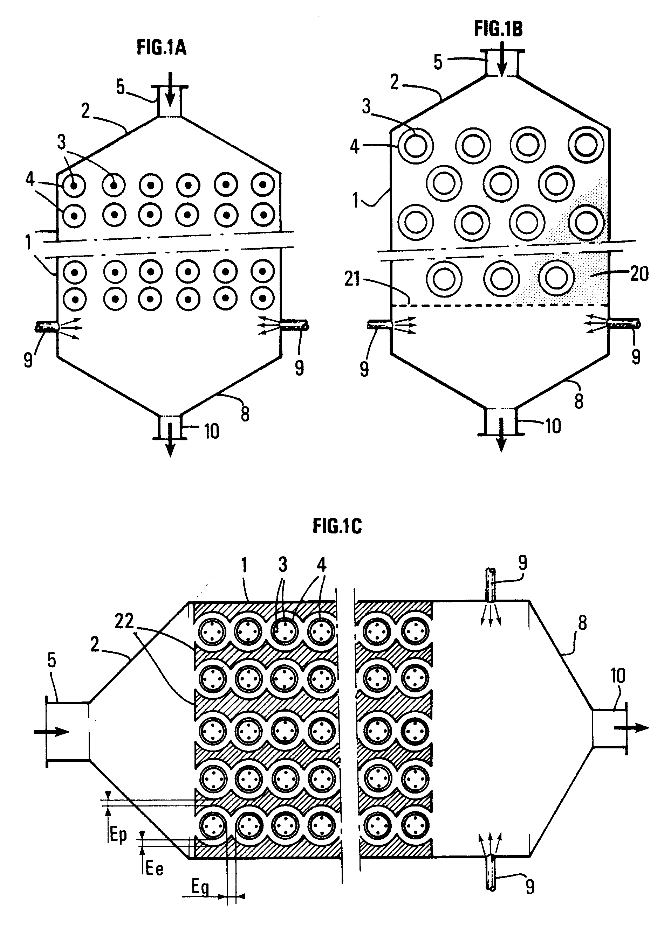

Dans cette forme de réalisation, les échanges convectifs entre le gaz process et les parois sont largement augmentés et ils peuvent être encore améliorés en imposant au gaz des vitesses importantes et en créant des zones de turbulence. L'augmentation de la vitesse des gaz peut par exemple être obtenue en utilisant des parois dont la forme favorise cette augmentation de vitesse et l'apparition de zones de turbulence. Des parois de forme particulières sont représentées à titre non limitatif sur la figure 1C.

Les parois sont habituellement en matériau réfractaire. Tout matériau réfractaire peut être utilisé pour réaliser les parois et on peut citer à titre d'exemples non limitatifs la zircone, le carbure de silicium, la mullite et divers bétons réfractaires.

Etant donné qu'il n'est nullement nécessaire d'avoir une étanchéité au niveau des parois, puisque la composition du gaz est pratiquement identique de chaque côté des parois, cette réalisation n'induit qu'une augmentation minime du coût du four. En effet, d'une part il n'est pas nécessaire d'avoir des parois spécialement épaisses, ni une réalisation particulièrement complexe, d'autre part les dimensions globales du four augmentent peu car l'essentiel de la largeur de ce four est due à la largeur des gaines. A titre d'exemple, les gaines peuvent avoir une largeur de l'ordre de 150 mm, pour une épaisseur de paroi ayant une valeur de l'ordre de 50 mm, ce qui ne provoque qu'une augmentation de largeur globale du four de l'ordre de 30 %.

Un avantage supplémentaire de cette réalisation comportant des parois est de permettre une réalisation plus simple du four, les parois verticales permettant, outre l'amélioration du tranfert thermique par convection, de supporter la voute du four.

Selon l'une des caractéristiques de l'invention, les résistances électriques qui fournissent de la chaleur à la zone de chauffage sont alimentées de façon indépendante en énergie électrique soit isolément, soit par petits groupes, de façon à définir des sections de chauffage le long de la zone de chauffage et à pouvoir ainsi moduler la quantité d'énergie fournie tout au long de cette zone.

La zone de chauffage est habituellement composée de 2 à 20 sections de chauffage, de préférence de 5 à 12 sections. Dans la première partie de cette zone, le mélange gazeux renfermant du méthane, préalablement chauffé à environ 750°C, est habituellement porté à une température au plus égale à environ 1500°C, et avantageusement entre 1000 et 1300°C (le début de la zone de chauffage est situé à l'endroit où la charge est introduite).

La modulation de ces sections de chauffage est réalisée de façon classique ; les éléments résistants correspondant aux sections précitées sont en général alimentés par des ensembles modulateurs à thyristors. Des transformateurs permettent éventuellement d'adapter les tensions à priori, alors que les modulateurs permettent le réglage fin et continu de la puissance injectée.

Afin de permettre la régulation de l'ensemble, chaque section de chauffage peut être munie d'une canne pyrométrique à thermocouple adaptée au niveau de température ; ces cannes sont disposées dans les espaces où circule la charge, les informations sont tranmises au régulateur qui commande le modulateur à thyristor.

Dans la première partie de la zone de chauffage, l'énergie électrique sert presque exclusivement à porter le mélange réactionnel de sa température initiale (par exemple environ 750°C) à la température où les réactions endothermiques de couplage deshydrogénant du méthane ont lieu ( par exemple environ 1200°C). C'est donc au début de la deuxième partie de la zone de chauffage qu'il est nécessaire de founir l'énergie maximum au milieu réactionnel, ce qui est facilement réalisé par la modulation d'une ou plusieurs sections de chauffage.

La longueur de la première partie de la zone de chauffage représente habituellement de 20 à 80 % de la longueur totale de la zone de chauffage,avantageusement de 30 à 70 %.

L'énergie électrique fournie à cette première partie de la zone de chauffage est telle qu'elle génère un fort gradient de température, habituellement d'environ 0,5 à environ 25°C/cm, et avantageusement d'environ 1 à environ 20°C/cm.

Dans la deuxième partie de la zone de chauffage, on module l'énergie électrique fournie aux différentes sections de chauffage de cette zone de façon à ce que la variation de température tout au long de cette zone soit faible, habituellement inférieure à environ 50°C (+ ou - 25°C autour de la valeur de la consigne) et avantageusement inférieure à environ 20°C.

Par ailleurs, l'utilisation de différentes sections transversales de chauffage, indépendantes les unes des autres, permet d'apporter, au niveau de la deuxième partie de la zone de chauffage, le maximum d'énergie thermique à l'endroit où s'effectue la plus grande partie des réactions endothermiques, et de maintenir dans le reste de la zone de chauffage une température quasi uniforme.

La longueur de la zone de chauffage est habituellement d'environ 50 à environ 90 % de la longueur totale de la zone réactionnelle.

On obtient, notamment dans les conditions de chauffage ci-dessus, un flux thermique très important à un niveau de température élevé. Ceci implique habituellement un choix particulier pour le matériau constitutif des résistances qui doit, outre le fait d'être résistant à l'atmosphère réductrice dans les conditions de température du fonctionnement, être capable de délivrer une puissance par unité de surface relativement importante. A titre d'exemple de matériau utilisable pour la réalisation des résistances, on peut citer le bisiliciure de molybdène (MoSi₂). Ces éléments chauffants en bisiliciure de molybdène présentent de nombreux avantages lors de leur utilisation à haute température :
- ils acceptent une charge (puissance émise par unité de surface) importante qui peut aller jusqu'à 20 W/cm²,
- ils peuvent travailler à très haute température,
- ils accusent dans le temps un vieillissement négligeable,
- ils supportent facilement des atmosphères réductrices à des températures élevées.

Dans le procédé de l'invention, la zone de chauffage est suivie d'une zone de refroidissement (ou trempe) de façon à abaisser très rapidement la température des effluents de la zone de chauffage vers environ 300°C par exemple.

Selon un mode de réalisation, on procède à une trempe directe ; les effluents de réaction quittent la zone de chauffage et sont très rapidement refroidis par une mise en contact direct avec un fluide de refroidissement qui est injecté dans les effluents au moyen d'au moins un injecteur, habituellement en matériau céramique, disposé à la périphérie du réacteur. On peut utiliser, comme fluide de refroidissement des gaz de pétrole liquifiés, du propane, des huiles hydrocarbonées ou de l'eau. Le propane est le gaz de trempe préféré car il peut également être partiellement craqué et contribuer ainsi à la formation de produits tels que l'éthylène. Les effluents totaux résultant du mélange sont ensuite recueillis et séparés.

Selon un mode de réalisation préféré, les effluents de réaction issus de la zone de chauffage sont refroidis par mise en contact indirect avec un fluide de refroidissement, par exemple en faisant circuler ledit fluide dans des conduits étanches à l'intérieur de la zone de refroidissement.

L'ensemble de ces caractéristiques permet d'obtenir, grâce à ce procédé, une conversion thermique du méthane en acétylène, éthylène et produits benzèniques s'effectuant avec un bon taux de conversion et une sélectivité, en ces produits, élevée.

Les charges hydrocarbonées utilisables dans le cadre de l'invention sont des charges gazeuses dans les conditions normales de température et de pression, comprenant habituellement un pourcentage en volume de méthane d'au moins 10 %, par exemple de 10 à 99 %, le plus souvent de 20 à 99 % et préférentiellement de 30 à 80 %. Le reste de la charge comprend, comme précisé ci-avant, dans presque tous les cas une proportion en volume d'hydrogène allant le plus souvent de 1 à 90 %. Elle peut également comprendre d'autres gaz tels que, par exemple, des hydrocarbures aliphatiques, saturés ou non, comprenant un nombre d'atomes égal ou supérieur à 2 comme l'éthylène, l'éthane, le propane ou le propylène. Elle peut aussi comprendre de l'azote, du dioxyde de carbone ou du monooxyde de carbone.

On peut, en restant dans le cadre de l'invention, rajouter aux charges définies ci-avant de la vapeur d'eau de dilution ; le rapport pondéral de la vapeur d'eau de dilution à la charge hydrocarbonée est en général de l'ordre de 0,1 : 1 à 1 : 1.

Les charges à traiter ont un temps de séjour dans la zone réactionnelle habituellement d'environ 2 millisecondes à environ 1 seconde et de préférence d'environ 30 à environ 400 millisecondes.

Le gaz contenant de l'hydrogène que l'on introduit dans les gaines entourant les résistances peut être de l'hydrogène sensiblement pur, de l'hydrogène industriel ou un mélange d'hydrogène avec un autre gaz inerte tel que par exemple de l'azote, de l'hélium, de l'argon, de la vapeur d'eau ou du gaz carbonique. On utilise de préférence de l'hydrogène pur ou industriel ou un mélange d'hélium et d'hydrogène ou un mélange d'argon et d'hydrogène ou un mélange de vapeur d'eau et d'hydrogène contenant habituellement au moins 5 % en volume d'hydrogène et de préférence au moins 10 %. Lorsqu'on utilise un mélange d'azote et d'hydrogène, celui-ci contient habituellement au moins 25 % en volume d'hydrogène et de préférence au moins 50 %.

La perméabilité des gaines doit être suffisante pour permettre, au moins en certains points, la diffusion d'au moins une partie de l'hydrogène, contenu dans le gaz introduit dans l'espace des résistances, vers l'espace process. On ne sortirait pas du cadre de l'invention dans le cas où la perméabilité des gaines serait telle qu'elle permette la diffusion de l'ensemble des composes gazeux, contenu dans le gaz introduit dans l'espace des résistances, vers l'espace process. Cette perméabilité peut résulter d'une étanchéité sur chaque gaine, réalisée volontairement de façon imparfaite et/ou de l'utilisation d'un matériau constitutif des gaines ayant une porosité ouverte permettant le passage de l'hydrogène, c'est-à-dire en d'autres termes un matériau perméable à l'hydrogène. Le plus souvent, il est recommandé d'utiliser un matériau perméable.

Ainsi, selon une réalisation préférée de l'invention, les gaines, isolant les moyens de chauffage électrique du contact direct avec le mélange gazeux renfermant du méthane, sont constituées d'un matériau poreux dont la porosité est suffisante pour permettre la diffusion de l'hydrogène à travers lesdites gaines. Ces gaines sont ainsi, de préférence, fabriquées en matière céramique poreuse présentant une porosité ouverte d'au moins environ 1 % et d'au plus environ 40 % en volume par rapport au volume de la paroi, et habituellement d'environ 5 % à environ 30 %.

L'utilisation de l'hydrogène sensiblement pur, qui diffuse au moins en partie vers l'espace process, procure plusieurs avantages. Il ne complique pas les séparations en aval du four à pyrolyse puisque le gaz à craquer est habituellement un mélange de méthane ou gaz naturel et d'hydrogène dans une proportion en volume de préférence de 10 à 80 % d'hydrogène et le plus souvent de 30 à 70 %.

L'introduction d'hydrogène tout le long du four de pyrolyse permet de diminuer la taille globale du four. En effet, si l'on vise en sortie une certaine proportion d'hydrogène dans le gaz craqué, en entrée du four celle-ci sera moindre et pour un même temps de séjour à respecter, le volume réactionnel sera moindre, donc la taille du four également. Mais, de plus, cette réalisation amènera une proportion croissante d'hydrogène le long du four de pyrolyse, ce qui représente un avantage du point de vue de la cinétique de craquage et de la stabilité des produits car, en début de four, une trop grande quantité d'hydrogène inhiberait par trop les réactions de craquage mais, en fin de four, alors qu'il y a une quantité notable de produits formés, notamment d'éthylène et d'acétylène, il est avantageux d'avoir une quantité d'hydrogène plus importante afin d'éviter la formation de coke. De l'hydrogène penétrant dans la zone process au niveau de la réalisation de chaque étanchéité (au moins une par gaine) sur chaque gaine protégeant les résistances, et/ou à travers la paroi des gaines par diffusion, on aura donc bien l'effet désiré.

Par ailleurs, selon la présente invention, étant donné qu'il n'est plus souhaitable de rechercher des étanchéités aussi parfaites que possible entre l'espace process et l'espace des résistances, on diminue le coût de réalisation du four tout en diminuant également les contraintes thermo-mécaniques au niveau des raccords des gaines, ce qui augmente la fiabilité de l'ensemble du dispositif.

Un autre avantage réside dans le choix qu'il est alors possible de faire quant aux gaines de protection des résistances délimitant l'espace process de l'espace des résistances. En effet, lorsque l'on utilise comme gaz d'étanchéité de l'azote ou du CO₂, comme on l'a mentionné à plusieurs reprises, il est nécessaire pour de multiples raisons de limiter la consommation de ce gaz, c'est-à-dire la fuite dudit gaz hors de l'espace des résistances vers l'espace process. Comme on l'a vu, cela est réalisé par la recherche d'une étanchéité aussi parfaite que possible, au niveau notamment des jonctions gaines-reste du four. Cela est aussi réalisé par l'utilisation de gaines en céramiques, et en particulier en carbure de silicium, aussi étanches que possibles, c'est-à-dire de très grande qualité et donc de très grand prix.

Il est en effet bien connu de l'homme du métier qu'il existe de nombreuses variétés de céramique, et notamment de carbure de silicium, qui proviennent de qualité de poudre constitutive et de conditions de frittage très différentes. Sans vouloir entrer dans le détail, on peut cependant noter qu'un des critères de qualité est lié à la plus faible rémanence de porosité après frittage. Il est bien connu que si une partie de cette porosité se trouve être fermée, c'est-à-dire sans effet sur l'étanchéité globale du matériau, une autre partie, non négligeable, surtout pour le carbure de silicium le plus courant, est une porosité ouverte et que notamment il y a, à haute température, diffusion de l'hydrogène à travers ce matériau. On comprend donc bien que lorsque l'on utilise comme gaz d'étanchéité un gaz tel que l'azote ou le CO₂, on doit utiliser un carbure de silicium de très haute qualité, dont la densité est proche de la densité théorique, donc exempt quasiment de porosité ouverte, pour éviter , d'une part une fuite dudit gaz de la zone des résistances vers l'espace process, et d'autre part, la différence de pression partielle de l'hydrogène étant positive dans le sens process - résistances, une diffusion de l'hydrogène contenu dans le gaz process vers l'espace des résistances.

Au contraire, dans le cadre de la présente invention, ce qui était un inconvénient rédhibitoire devient un avantage déterminant.

L'utilisation de gaines en céramique, notamment en carbure de silicium, de qualité moyenne, comportant une porosité ouverte d'au moins environ 1 % en volume (par exemple d'environ 20 % en volume), est ainsi non seulement possible mais même souhaitable, ce qui abaisse le coût de réalisation du four. Par ailleurs, l'existence même de cette porosité ouverte crée en surface de la gaine en céramique côté espace process une pression partielle d'hydrogène isolant en quelque sorte la surface de la céramique du gaz process ce qui, sans vouloir être lié par une quelconque théorie, explique la réduction notable de la formation de coke puisque celui-ci se forme habituellement pour l'essentiel à la surface des gaines et qu'au contraire les produits formés se trouveront dans une atmosphère locale plus riche en hydrogène, donc moins favorable à la formation de coke.

Par porosité ouverte, on désigne dans la description de la présente invention la porosité constituée de microcavités incluses dans les pièces en céramiques massives considérées, l'adjectif ouvert signifiant qu'il y a libre passage d'une part entre la plupart desdites microcavités, et d'autre part entre lesdites microcavités et les surfaces internes et externes des pièces considérées ; la notion de libre passage doit aussi être considérée en fonction de la nature du milieu et des conditions physiques dans lesquelles se trouve la céramique. En particulier, pour des molécules de petites tailles comme l'hydrogène ou l'hélium, le libre passage sera facile, d'autant plus d'ailleurs s'il l'on a une différence de pression entre les deux surfaces de la pièce en céramique. Dans ce cas, la pièce est dite perméable, à l'hydrogène par exemple, ou non étanche.

Par porosité fermée, on désigne dans la description de la présente invention la porosité constituée de microcavités ne communiquant pas avec la surface de la pièce. Dans ce cas à, cette porosité fermée ne provoque qu'une diminution globale de la densité de la pièce.

L'invention a également pour objet le dispositif pour la mise en oeuvre du procédé. Ce dispositif peut également être utilisé pour la réalisation d'autres réactions endothermiques s'effectuant habituellement à des températures supérieures à environ 600°C, et par exemple d'environ 700 à environ 1450°C, avec des temps de séjour de l'ordre de 2 millisecondes à 1 seconde.

Plus particulièrement, l'invention concerne un dispositif pour la mise en oeuvre du procédé comprenant un réacteur (1) de forme allongée selon un axe, de préférence à section carrée ou rectangulaire, comportant à une première extrémité des moyens d'alimentation en mélange gazeux, à l'extrémité opposée des moyens d'évacuation des effluents produits et entre ces deux extrémités des moyens d'alimentation en fluide de refroidissement, ledit réacteur comprenant dans une première partie (côté première extrémité) une pluralité de moyens de chauffage électrique (3) entourés de gaines (4), lesdits moyens sensiblement parallèles entre eux étant disposés en nappes sensiblement parallèles, perpendiculaires à l'axe du réacteur de façon à définir entre les gaines et/ou les nappes formées par ces gaines des espaces ou passages pour la circulation des mélanges gazeux et/ou des effluents, lesdits moyens de chauffage et lesdites gaines étant adaptés à chauffer lesdits passages par section transversales successives, indépendantes et sensiblement perpendiculaires à l'axe du réacteur, ledit réacteur comportant en outre des moyens d'asservissement et de modulation de chauffage reliés auxdits moyens de chauffage, et comportant dans une deuxième partie (8) (côté extrémité opposée) contiguë à la première partie des moyens de refroidissement (9) des effluents reliés auxdits moyens d'alimentation en fluide de refroidissement, ledit dispositif étant caractérisé en ce qu'il comprend des moyens d'introduction, à une pression appropriée, d'un gaz contenant de l'hydrogène, a l'intérieur des gaines (4) et en ce que lesdites gaines ont une perméabilité suffisante pour permettre, au moins en certains points, la diffusion d'au moins une partie de l'hydrogène depuis l'intérieur desdites gaines vers l'extérieur desdites gaines, cet hydrogène se diluant alors dans ledit mélange gazeux.

Les moyens d'introduction, à une pression appropriée, du gaz sont ceux connus de l'homme du métier. Ils peuvent comprendre par ailleurs des moyens de régulation et de contrôle des pressions régnant à l'intérieur et à l'extérieur desdites gaines.

Lesdits moyens de refroidissements sont des moyens adaptés à refroidir par contact direct ou par contact indirect les effluents quittant la première zone.

Les gaines entourant les résistances, habituellement de façon non jointive, peuvent être disposées de façon superposée ou en quinconce et peuvent former en projection transversale un faisceau à pas triangulaire ou carré.

Le nombre total de nappes des moyens de chauffage et le nombre des moyens de chauffage par nappe ne sont pas déterminants dans le procédé ; ils sont évidemment fonction de la dimension, des moyens de chauffage, des gaines qui les entourent et, lorqu'elle existent, des parois séparant les nappes. Les éléments de chauffage peuvent être identiques entre eux ou différents, tant par leurs dimensions que par leur puissance de chauffage.

Le nombre d'éléments chauffant détermine la puissance électrique maximum disponible pour un volume réactionnel donné et influe également sur le temps de séjour de la charge ; il sera choisi en fonction du débit de charge admissible, compte tenu de ces paramètres.

On peut, dans le cadre de la présente invention, réaliser l'ensemble du réacteur zone de chauffage et zone de trempe soit sous forme monobloc, soit encore par juxtaposition jointive de divers éléments de forme identique, qui sont assemblés entre eux par tout moyen utilisable comme, par exemple, à l'aide de brides.

Les moyens de chauffage électrique utilisables dans le cadre de la présente invention sont de préférence des résistances chauffantes susceptibles d'être utilisées, en atmosphère globalement réductrice, jusqu'à des températures de l'ordre de 1500°C ; on préfère utiliser des résistances en bisiliciure de molybdène, par exemple des résistances en épingle.

Les gaines qui entourent les résistances, de façon à éviter un contact direct entre les mélanges gazeux de la charge et les résistances, sont, de préférence, de forme tubulaire. Ces gaines en matériau réfractaire sont le plus souvent en céramique. On peut utiliser des céramiques comme la mullite, la cordiérite, le nitrure de silicium, le carbure de silicium, la silice ou l'alumine ; le carbure de silicium est le matériau préférentiellement choisi car il présente une bonne conductivité thermique. Dans le cas où les nappes sont séparées par des parois, le matériau choisi pour réaliser ces parois peut être le même que celui utilisé pour les gaines, mais il est souvent différent, en particulier pour des questions de coût de fabrication du four.

La distance qui sépare les éléments chauffants des gaines est fonction de la section de l'élément chauffant. Pour des moyens de chauffage dont le diamètre maximal du cercle les englobant est égal à d, on utilise des gaines habituellement tubulaires ou cylindriques de diamètre D habituellement d'environ 1,2xd à environ 8xd, et le plus souvent d'environ 1,5xd à environ 4xd.

Les éléments de chauffage sont disposés en nappes parallèles sensiblement perpendiculaires au sens d'écoulement de la charge (gaz process), préférentiellement sensiblement alignés, de façon à ce que la distance qui sépare deux gaines voisines soit la plus réduite possible, tout en tenant compte des impératifs de perte de charge admissible ; la distance entre les gaines de deux nappes voisines ou celle entre les gaines d'une nappe et la paroi la plus proche dans le cas où les nappes sont séparées par des parois est habituellement la même que celle entre deux gaines consécutives dans une nappe donnée. Cette distance sera habituellement telle que les passages formés entre les gaines ou entre les gaines et la paroi plus proche, passages dans lesquels circule le mélange gazeux renfermant du méthane auront une dimension d'environ 1 à environ 100 mm, et le plus souvent d'environ 5 à environ 40 mm.

Selon un mode de réalisation de l'invention, les espaces libres ou passages définis ci-avant, destinés à la circulation du gaz process, sont au moins partiellement occupés par des garnissages, habituellement en céramique, préférentiellement conducteurs de la chaleur. On peut ainsi, pour un type de réacteur donné, diminuer le temps de séjour de la charge dans ce réacteur tout en homogénéisant l'écoulement du mélange gazeux et en répartissant mieux la chaleur dissipée. Ces garnissages peuvent avoir des formes diverses et se présenter par exemple sous forme d'anneaux ( anneaux de Raschig, de Lessing ou de Pall), de selles (selles de Berl), de barreaux, de tubes cylindriques fermés.

L'invention sera mieux comprise par la description de quelques modes de réalisation, donnés à titre purement illustratif mais nullement limitatif qui en sera faite ci-après à l'aide des figures annexées, sur lesquelles les organes similaires sont désignés par les même chiffres et lettres de référence.
- Les figures 1A, 1B et 1C réprésentent une coupe longitudinale d'un réacteur suivant un plan perpendiculaire à l'axe des gaines. Dans le cas de la figure 1B, ce réacteur contient un garnissage. Dans le cas de la figure 1C, ce réacteur comporte des parois séparant les nappes successives de gaines.
- Les figures 1D et 1E réprésentent une coupe longitudinale d'un réacteur suivant l'axe des gaines.
- La figure 2 illustre un détail de réalisation de la zone de chauffage dans un plan identique à celui des figures 1D et 1E.

Sur la figure 1A, on a représenté, selon un mode de réalisation, un réacteur (1) vertical de forme allongée et de section rectangulaire, comprenant un distributeur (2) permettant d'alimenter par un orifice d'entrée (5) le réacteur en mélange gazeux réactionnel. Ce dernier, qui contient par exemple 50 % de méthane, a été préchauffé dans une zone de préchauffage conventionnelle, non représentée sur la figure, de préférence par convection. Le réacteur comprend une pluralité de moyens de chauffage électrique (3) entourés de gaines (4), disposées en nappes parallèles et formant dans un plan (plan de la figure) un faisceau à pas carré. Ces nappes définissent des sections de chauffage transversales sensiblement pependiculaires à l'axe du réacteur défini selon la direction d'écoulement de la charge.

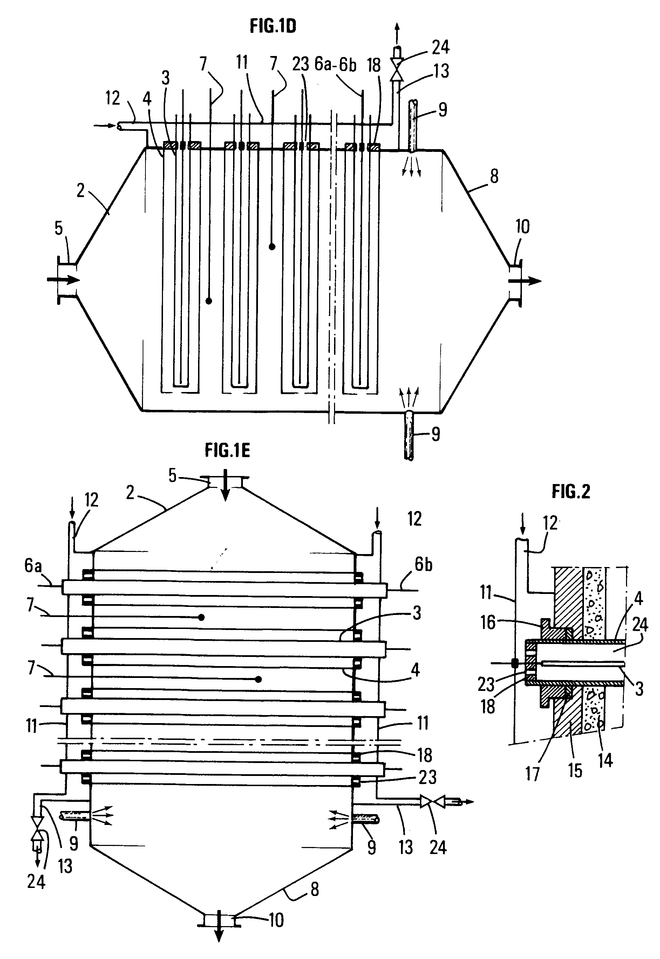

Ces sections de chauffage sont alimentées en énergie électrique, de façon indépendante, grâce à une paire d'électrodes (6a, 6b sur les figures 1D et 1E), des sondes pyromètriques à thermocouple (7 sur les figures 1D et 1E) sont logées dans les espaces où circule la charge entre les gaines (4) et permettent de réguler automatiquement la température de chaque section de chauffage, par un dispositif classique de régulateur et de modulateur non représenté sur la figure.

Dans la première partie de la zone de chauffage, les gaines sont chauffées de façon à ce que la température de la charge passe rapidement de 750°C (température de préchauffage) à 1200°C environ ; cette zone de chauffage progressif représente en général environ 65 % de la longueur totale de la zone de chauffage ; le mélange gazeux circule ensuite dans la deuxième partie de la zone de chauffage où l'on maintient généralement la température à une valeur constante sensiblement égale à celle atteinte à la fin de la première zone de chauffage, soit en général 1200°C environ. A cet effet, on module la puissance électrique fournie à plusieurs sections de chauffage qui constituent la deuxième partie de la zone de chauffage ; on parvient ainsi à obtenir une variation de température ne dépassant pas environ 10°C autour de la valeur de la consigne. La longueur de cette deuxième zone de chauffage représente environ 35 % de la longueur totale de la zone de chauffage.

A la sortie de la zone de chauffage, les effluents de la réaction sont refroidis dans une zone de refroidissement (8). Ils sont mis en contact avec un agent de trempe tel que du propane introduit par l'intermédiaire d'injecteurs (9), de trempe, disposés à la périphérie du réacteur (1) et reliés à une source extérieure de propane non représentée. L'ensemble des gaz effluents est refroidi à une température d'environ 500°C et recueilli par un orifice de sortie (10) à l'extrémité de la zone réactionnelle (1).

Selon un autre mode non illustré, les effluents peuvent être refroidis en circulant à travers des conduits étanches disposés dans la zone (8) par lesquels s'écoule l'agent de trempe, ces conduits étant reliés à la source extérieure de l'agent de trempe.

Selon le mode de réalisation schématisé sur la figure 1B le réacteur, identique à celui schématisé sur la figure 1A, comporte dans l'espace où circule la charge un garnissage (20), avantageusement en matière céramique, qui est retenu par une grille (21) à l'extrémité de la zone de chauffage. Les gaines (4) sont disposées en nappes parallèles et forment dans un plan (plan de la figure) un faisceau à pas triangulaire (disposition en quinconce).

Sur la figure 1C, on a représenté, selon un mode de réalisation, un réacteur (1) horizontal de forme allongée et de section rectangulaire, qui ne diffère du réacteur représenté sur la figure 1A que par le fait qu'il est sensiblement horizontal, qu'il comporte des gaines disposées en nappes parallèles et formant dans un plan (plan de la figure) un faisceau à pas carré, et en ce que ces nappes sont séparées les unes des autres par des parois (22) avantageusement en matière céramique. Ces parois ont une forme, adaptée à créer des turbulences, comportant des alvéoles au niveau de chaque gaine(4).

La figure 1D représente, pour un réacteur horizontal, les mêmes éléments que ceux décrits en liaison avec la figure 1A ; on a représenté, de plus, un boîtier de protection (11) comportant un orifice (12) par lequel on introduit le gaz contenant de l'hydrogène et un orifice (13) muni d'une vanne (24) permettant de réguler le flux du gaz contenant de l'hydrogène. Ce boîtier (11) est fixé sur l'armature métallique du réacteur (1) et entoure l'ensemble des résistances électriques et des gaines les contenant, à l'exception de l'extrémité des résistances électriques par où se fait l'alimentation en énergie électrique. Les résistances (3), en épingle, sont positionnées dans les gaines (4) à l'aide de rondelles (18), par exemple en fibre céramique, comportant des passages (23) permettant au gaz contenant de l'hydrogène de pénétrer dans l'espace compris entre les résistances et les gaines.

La figure 1E représente les mêmes éléments que ceux décrits en liaison avec la figure 1A ; on a représenté, de plus, les boîtiers de protection (11) munis d'orifice (12) et (13) permettant la circulation dans les boîtiers du gaz contenant I'hydrogène qui pénètre dans l'espace des résistances par les orifices (23) des rondelles (18) assurant le positionnement des résistances. Les orifices (13) sont munis de vannes (24) permettant une régulation plus facile du flux du gaz contenant de l'hydrogène. Ces boîtiers (11) sont fixés sur l'armature métallique du réacteur et entourent l'ensemble des résistances électriques et des gaines les contenant, à l'exception de l'extrémité des résistances électriques par où se fait l'alimentation en énergie électrique. La circulation du gaz contenant de l'hydrogène est habituellement effectuée en légère surpression par rapport à la pression du gaz process au sein du réacteur, assurant ainsi une atmosphère parfaitement contrôlée et une meilleure diffusion de l'hydrogène contenu dans ce gaz vers l'espace process.

La pression pourrait être pratiquement égale à celle du gaz process et, dans ce cas, comme dans le cas d'une surpression globale, il est habituellement préférable que la pression partielle de l'hydrogène soit légèrement plus élevée dans l'espace des résistances que dans l'espace process, de manière à être certain que l'hydrogène diffuse bien depuis l'espace des résistances vers l'espace process. La différence des pressions partielles de l'hydrogène sera le plus souvent telle que la pression partielle de l'hydrogène au sein du gaz contenu dans l'espace des résistances soit supérieure d'au moins 0,1 % et de préférence d'au moins 1 % à celle de l'hydrogène contenu dans le gaz process. La différence de pression absolue entre l'espace des résistances et l'espace process, ou surpression, sera de préférence telle que la pression dans l'espace des résistances soit supérieure d'au moins 0,1 % et le plus souvent d'au moins 1 % à la pression dans l'espace process. Il n'est pas nécessaire d'avoir une très grande surpression et le plus souvent la pression dans l'espace des résistances reste inférieure à 2 fois la pression dans l'espace process.

La figure 2 représente un détail d'un mode de réalisation de la zone de chauffage suivant l'invention. On utilise comme moyen de chauffage électrique des résistances (3) de forme cylindique. Ces résistances comportent à chacune de leur extrémité des zones froides et une partie de la zone centrale qui est la zone chaude représentant par exemple environ 68 % de la longueur totale.

On réalise un réacteur de section rectangulaire, dont les parois sont constituées de béton réfractaire isolant (14) et par une armature métallique (15). On perce dans deux parois latérales opposées un trou circulaire, dans lequel on fait passer une gaine (4), par exemple en céramique, de diamètre double de celui de la résistance électrique (3). La gaine (4) est positionnée au moyen d'un système presse étoupe (16) agissant dans une gorge au niveau de l'armature métallique sur une tresse en matière réfractaire (17), par exemple une tresse en matière céramique. Le positionnement de la résistance (3) dans la gaine (4) est effectué au moyen de rondelles (18), par exemple en fibre céramique, comportant des orifices (23) permettant le passage du gaz contenant de l'hydrogène introduit dans le boîtier (11) par le conduit (12) dans l'espace des résistances (24).

La zone chaude de la résistance (3) est positionnée de façon à ce qu'elle ne pénètre pas dans l'orifice de passage à travers la paroi de béton isolant. Il n'est pas indispensable d'utiliser une tresse (17) au niveau du presse étoupe puisque celui-ci a, dans le cadre de l'invention, le role de moyen de positionnement et qu'il n'a pas pour but principal d'assurer une étanchéité aussi parfaite que possible entre l'intérieur et l'extérieur du réacteur. Ce presse étoupe peut d'ailleurs avantageusement être remplacé par un moyen plus simple de positionnement des gaines tel que par exemple des simples rondelles en matériau réfractaire.

On dispose ainsi d'un certain nombre de résistances chauffantes gainées dans des parois, par exemple en matière céramique, par rangées horizontales successives, ces rangées étant de préférence alignées de façon à ce que, sur les parois latérales du four, elles forment un faisceau à pas carré ou rectangulaire. Un boîtier (11), dont dépassent seulement les extrémités des résistances et/ou leur alimentation électrique (6), est parcouru par un courant de gaz contenant de l'hydrogène.

### Exemple 1

On utilise un réacteur horizontal à trempe indirecte, de longueur totale de 6,1 mètre et de section rectangulaire de 1,4x3,2 m. Les moyens de chauffage de ce réacteur sont contitués par des résistances électriques en épingle, de marque KANTHAL, en bisiliciure de molybdène (MoSi₂) de type SUPERKANTHAL ; ces résistances sont entourées de gaines en céramique, disposées concentriquement par rapport au centre du cercle englobant les résistances.

Ces gaines sont en carbure de silicium fabriqué par la société NORTON, elles sont de type KRYSTON et ont une porosité ouverte de 15 % en volume. Chaque gaine, fermée à une extrémité, entoure 2 résistances en épingle (Figure 1D). Ces gaines sont disposées perpendiculairement au sens de circulation de la charge (verticalement), en nappes parallèles, et forment en projection perpendiculaire un faisceau à pas carré. La longueur de chaque branche de l'épingle de la résistance électrique est de 1,4 m et le diamètre de la résistance est de 9 mm. Les gaines en céramique ont une longueur de 1,4 m, un diamètre extérieur de 150 mm et un diamètre intérieur de 130 mm ; la distance séparant deux gaines voisines est de 10 mm.

La première partie de la zone de chauffage, longue de 3,7 m, comprend 23 nappes de résistances, chaque nappe comprenant 20 gaines ; dans cette zone, la charge, préchauffée à 800°C, est portée à 1200°C. Cette zone est régulée thermiquement par l'intermédiaire de thermocouples disposés dans les espaces où circule la charge.

La deuxième partie de la zone de chauffage, adjacente à ladite première partie, est longue de 2,4 m ; elle est constituée de 15 nappes de 20 gaines, disposées de la même façon que dans la première partie de la zone de chauffage. Cette zone est constituée par 5 sections de chauffage, régulées indépendamment, permettant d'assurer le maintien de la température dans cette zone à 1200°C plus ou moins 10°C.

Les gaz effluents sont refroidis dans un premier temps à 800°C par échange indirect avec les gaz de la charge ; d'autres échangeurs de température permettent ensuite d'abaisser leur température à 300°C environ.

On utilise comme charge du méthane dilué avec de l'hydrogène dans un rapport volumique de 1 : 1. Ce mélange est préchauffé à 800°C et craqué dans le réacteur décrit ci-avant. La pression absolue du mélange de gaz dans le réacteur est maintenue sensiblement constante et égale à 0,125 MPa. On introduit dans l'espace des résistances de l'hydrogène sensiblement pur de manière à obtenir et à maintenir dans cet espace une pression absolue sensiblement constante et égale à 0,130 MPa.

Après refroidissement à température ambiante, pour 200 moles de mélange équivolumique de méthane et d'hydrogène, on obtient les quantités suivantes de produits principaux :

| **PRODUITS** | **QUANTITES** |
|---|---|
| H₂ | 143 moles |
| CH₄ | 70 moles |
| C₂H₂ | 6 moles |
| C₂H₄ | 4 moles |
| Benzène | 0,75 moles |
| Coke | 54g |

### Exemple 2

On utilise un réacteur horizontal à trempe indirecte, de longueur totale de 4,3 mètres et de section rectangulaire de 1,4x3,7 m. Les moyens de chauffage de ce réacteur sont contitués par des résistances électriques en épingle, de marque KANTHAL, en bisiliciure de molybdène (MoSi₂) de type SUPERKANTHAL ; ces résistances sont entourées de gaines en céramique, disposées concentriquement par rapport au centre du cercle englobant les résistances.

Ces gaines sont en carbure de silicium fabriqué par la société NORTON, elles sont de type KRYSTON et ont une porosité ouverte de 15 % en volume. Chaque gaine, fermée à une extrémité, entoure 2 résistances en épingle (Figures 1C et 1D). Ces gaines sont disposées perpendiculairement au sens de circulation de la charge (verticalement), en nappes parallèles, et forment en projection perpendiculaire un faisceau à pas carré. La longueur de chaque branche de l'épingle de la résistance électrique est de 1,4 m et le diamètre de la résistance est de 9 mm. Les gaines en céramique ont une longueur de 1,4 m, un diamètre extérieur de 150 mm et un diamètre intérieur de 130 mm ; la distance Eg (Figure 1C) séparant deux gaines voisines est de 20 mm. Les nappes de gaines sont séparées par une paroi en béton réfractaire à base d'alumine électofondue. La distance Ee (Figure 1C) entre les gaines et les paroi ou dimension des passages est de 10 mm. Les parois ont dans leur partie la plus mince une épaisseur Ep (Figure 1C) de 15 mm. Le réacteur comporte ainsi 25 nappes de 20 gaines et 19 parois.

La première partie de la zone de chauffage, longue de 1,7 m, comprend 10 nappes de résistances, chaque nappe comprenant 20 gaines ; dans cette zone, la charge, préchauffée à 1000°C, est portée à 1200°C. Cette zone est régulée thermiquement par l'intermédiaire de thermocouples disposés dans les espaces où circule la charge.

La deuxième partie de la zone de chauffage, adjacente à ladite première partie, est longue de 2,55 m ; elle est constituée de 15 nappes de 20 gaines, disposées de la même façon que dans la première partie de la zone de chauffage. Cette zone est constituée par 3 sections de chauffage, régulées indépendamment, permettant d'assurer le maintien de la température dans cette zone à 1200°C plus ou moins 10°C.

Les gaz effluents sont refroidis dans un premier temps à 800°C par échange indirect avec les gaz de la charge ; d'autres échangeurs de température permettent ensuite d'abaisser leur température à 300°C environ.

On utilise comme charge du méthane dilué avec de l'hydrogène dans un rapport volumique de 1 : 1. Ce mélange est préchauffé à 1000°C et craqué dans le réacteur décrit ci-avant. La pression absolue du mélange de gaz dans le réacteur est maintenue sensiblement constante et égale à 0,125 MPa. On introduit dans l'espace des résistances de l'hydrogène sensiblement pur de manière à obtenir et à maintenir dans cet espace une pression absolue sensiblement constante et égale à 0,130 MPa.

Après refroidissement à température ambiante, pour 200 moles de mélange équivolumique de méthane et d'hydrogène, on obtient les quantités suivantes de produits principaux :

| **PRODUITS** | **QUANTITES** |
|---|---|
| H₂ | 142,5 moles |
| CH₄ | 70 moles |
| C₂H₂ | 6,3 moles |
| C₂H₄ | 4 moles |
| Benzène | 0,74 moles |
| Coke | 48g |

## Revendications

1. Procédé de conversion thermique du méthane en hydrocarbures de poids moléculaire plus élévé dans une zone de réaction, de forme allongée selon une direction, comprenant une zone de chauffage et une zone de refroidissement faisant suite à ladite zone de chauffage, dans lequel on fait circuler, dans la zone de chauffage, un mélange gazeux, renfermant du méthane, selon une direction d'écoulement sensiblement parallèle à la direction de la zone de réaction, ladite zone de chauffage comportant une pluralité de moyens de chauffage électrique disposés en nappes, sensiblement parallèles entre elles, formant en projection transversale un faisceau à pas triangulaire, carré ou rectangulaire, lesdits moyens de chauffage étant regroupés par sections successives tranversales sensiblement perpendiculaires à la direction de la zone de réaction, indépendantes entre elles et alimentées en énergie électrique de façon à déterminer au moins deux parties dans la zone de chauffage, la première partie permettant de porter la charge jusqu'à une température au plus égale à environ 1500°C, et la deuxième partie, subséquente de la première partie, permettant de maintenir la charge à une température sensiblement égale à la température maximum à laquelle elle a été portée dans ladite première partie, et dans lequel on refroidit les effluents de la zone de chauffage par introduction dans la zone de refroidissement d'un fluide de refroidissement, puis on recueille lesdits hydrocarbures de poids moléculaires plus élevés à l'extrémité de la zone réactionnelle, ledit procédé étant caractérisé en ce que les moyens de chauffage électrique sont isolés du contact direct avec le mélange gazeux renfermant du méthane par des gaines dans lesquelles on introduit un gaz contenant de l'hydrogène, lesdites gaines ayant une perméabilité appropriée et le gaz étant introduit à l'intérieur desdites gaines à une pression telle qu'il y a diffusion, au moins en' certains points, d'au moins une partie de l'hydrogène depuis l'intérieur desdites gaines vers l'extérieur desdites gaines.

2. Procédé selon la revendication 1 dans lequel on chauffe une première partie de la zone de chauffage jusqu'à une température maximale au plus égale à environ 1500°C de façon à réaliser un gradient thermique d'environ 0,5 °C/cm à environ 25 °C/cm sur une longueur d'environ 20 à environ 80% de la longueur totale de la zone de chauffage et dans lequel on chauffe la deuxième partie, subséquente de ladite première partie, de façon à ce que la variation de température tout au long de cette deuxième partie de la zone de chauffage soit inférieure à environ 50°C et de préférence inférieure à environ 20°C.

3. Procédé selon la revendication 1 ou 2 dans lequel la pression du gaz dans les gaines est supérieure d'au moins 0,1 % à la pression à l'extérieur desdites gaines.

4. Procédé selon l'une des revendications 1 à 3 dans lequel les gaines isolant les moyens de chauffage électrique du contact direct avec le mélange gazeux renfermant du méthane sont constituées d'un matériau poreux dont la porosité est suffisante pour permettre la diffusion de l'hydrogène à travers lesdites gaines.

5. Procédé selon la revendication 5 dans lequel les gaines sont en matière céramique poreuse présentant une porosité ouverte d'au moins environ 1 % et d'au plus environ 40 % en volume.

6. Procédé selon l'une des revendications 1 à 5 dans lequel les moyens de chauffage sont isolés du contact direct avec le mélange gazeux renfermant du méthane par des gaines cylindriques dont le diamètre est d'environ 1,2 à environ 4 fois le diamètre maximal du cercle englobant lesdits moyens de chauffage.

7. Procédé selon l'une des revendications 1 à 6 dans lequel la dimension des passages dans lesquels circulent le mélange gazeux renfermant du méthane est d'environ 1 à environ 100 mm.

8. Procédé selon l'une des revendications 1 à 7 dans lequel chaque nappe est séparée de la suivante par une paroi en matériau réfractaire.

9. Procédé selon l'une des revendications 1 à 7 dans lequel les moyens de chauffage électrique comportent des résistances en bisiliciure de molybdène.

10. Dispositif pour la mise en oeuvre du procédé selon l'une des revendications 1 à 9 comprenant un réacteur (1) de forme allongée selon un axe, de préférence à section carrée ou rectangulaire, comportant à une première extrémité des moyens d'alimentation en mélange gazeux, à l'extrémité opposée des moyens d'évacuation des effluents produits et entre ces deux extrémités des moyens d'alimentation en fluide de refroidissement, ledit réacteur comprenant dans une première partie (côté première extrémité) une pluralité de moyens de chauffage électrique (3) entourés de gaines (4), lesdits moyens sensiblement parallèles entre eux étant disposés en nappes sensiblement parallèles, perpendiculaires à l'axe du réacteur de façon à définir entre les gaines et/ou les nappes formées par ces gaines des espaces ou passages pour la circulation des mélanges gazeux et/ou des effluents, lesdits moyens de chauffage et lesdites gaines étant adaptés à chauffer lesdits passages par section transversales successives, indépendantes et sensiblement perpendiculaires à l'axe du réacteur, ledit réacteur comportant en outre des moyens d'asservissement et de modulation de chauffage reliés auxdits moyens de chauffage, et comportant dans une deuxième partie (8) (côté extrémité opposée) contiguë à la première partie des moyens de refroidissement (9) des effluents reliés auxdits moyens d'alimentation en fluide de refroidissement, ledit dispositif étant caractérisé en ce qu'il comprend des moyens d'introduction, à une pression appropriée, d'un gaz contenant de l'hydrogène, à l'intérieur des gaines (4) et en ce que lesdites gaines ont une perméabilité suffisante pour permettre, au moins en certains points, la diffusion d'au moins une partie de l'hydrogène depuis l'intérieur desdites gaines vers l'extérieur desdites gaines.

11. Dispositif selon la revendication 10 dans lequel les moyens de chauffage électrique sont entourés par des gaines cylindriques dont le diamètre est d'environ 1,2 à environ 4 fois le diamètre maximal du cercle englobant lesdits moyens de chauffage.

12. Dispositif selon la revendication 10 ou 11 dans lequel chaque nappe est séparée de la suivante par une paroi en matériau réfractaire sensiblement perpendiculaire à l'axe du réacteur, lesdites parois étant disposées de façon à définir entre elles et les nappes formées par les gaines entourant les moyens de chauffage des espaces ou passages pour la circulation des mélanges gazeux et/ou des effluents.

13. Dispositif selon l'une des revendications 10 à 12 dans lequel les passages entre les gaines et/ou entre les nappes et/ou entre les nappes et les parois ont une dimension d'environ 1 à environ 100 mm.

14. Dispositif selon l'une des revendications 10 à 13 dans lequel les passages sont au moins partiellement remplis de garnissage en matière céramique.

## Claims

1. A method of thermal conversion of methane to hydrocarbons of higher molecular weight in a reaction zone, of an elongated shape in one direction, comprising a heating zone and a cooling zone following said heating zone, wherein a gas mixture containing methane is circulated in the heating zone in a direction of flow substantially parallel with the direction of the reaction zone, said heating zone comprising a plurality of electric heating means arranged in substantially parallel sheets which form a bundle of triangular, square or rectangular pitch in transverse elevation, said heating means being grouped in successive cross sections substantially normal to the direction of the reaction zone, the sections being independent of one another and supplied with electricity so as to establish at least two parts in the heating zone, the first part enabling the charge to be brought to a temperature of no more than about 1500°C and the second part, which follows the first, enabling the charge to be kept at a temperature substantially equal to the maximum temperature to which it was brought in said first part, and wherein the effluent from the heating zone is cooled by letting a cooling fluid into the cooling zone, whereupon said hydrocarbons of higher molecular weight are collected at the end of the reaction zone, characterised in that the electric heating means are insulated from direct contact with the gas mixture containing methane by sheaths into which a gas containing hydrogen is fed, said sheaths having appropriate permeability and the gas being fed into said sheaths at a pressure such that at least part of the hydrogen is diffused from the inside of said sheaths to the outside, at least at certain points.

2. The method of Claim 1, wherein a first part of the heating zone is heated to a maximum temperature of about 1500°C so as to form a heat gradient of about 0.5°C/cm to about 25°C/cm over a length of about 20 to about 80% of the total length of the heating zone, and wherein the second part, following the first part, is heated so that the variation in temperature right along the second part of the heating zone is less than about 50°C and preferably less than about 20°C.

3. The method of Claim 1 or 2, wherein the pressure of the gas in the sheaths is at least 0.1% higher than the pressure outside them.

4. The method of any of Claims 1-3, wherein the sheaths insulating the electric heating means from direct contact with the gas mixture containing methane are made of a porous material, with sufficient porosity to enable hydrogen to be diffused through said sheaths.

5. The method of Claim 4 , wherein the sheaths are made of porous ceramic material with an open porosity of a minimum of about 1% and a maximum of about 40% by volume.

6. The method of any of Claims 1-5, wherein the heating means are insulated from direct contact with the gas mixture containing methane by cylindrical sheaths, the diameter of which is from about 1.2 to about 4 times the maximum diameter of the circle containing said heating means.

7. The method of any of Claims 1-6, wherein the dimension of the passages in which the gas mixture containing methane flows is from about 1 to about 100 mm.

8. The method of any of Claims 1-7, wherein each sheet is separated from the next one by a wall of refractory material.

9. The method of any of Claims 1-7, wherein the electric heating means comprise resistors made of molybdenum bisilicide.

10. An arrangement for applying the method of any of Claims 1-9, comprising a reactor 1 of elongated shape along one axis, preferably of square or rectangular cross section, comprising means for supplying with gas mixture at a first end, means for discharging the effluent produced at the opposite end, and means for supplying with cooling fluid between the two ends, said reactor comprising in a first part (towards the first end) a plurality of electric heating means 3 surrounded by sheaths 4, said means being substantially parallel and arranged in substantially parallel sheets perpendicular to the axis of the reactor, so as to define spaces or passages for the circulation of the gas mixtures and/or effluent between the sheaths and/or the sheets formed by the sheaths, said heating means and said sheaths being adapted to heat said passages by successive independent cross sections substantially normal to the axis of the reactor; said reactor further comprising heat control and heat modulating means connected to said heating means, and comprising in the second part 8 (towards the opposite end) adjoining the first part, means 9 for cooling effluent, connected to said means for supplying cooling fluid, characterised in that it comprises means for letting a gas containing hydrogen into the sheaths 4 at an appropriate pressure, and that said sheaths have sufficient permeability to enable at least part of the hydrogen to be diffused from the inside of said sheaths to the outside, at least at certain points.

11. The arrangement of Claim 10, wherein the electric heating means are surrounded by cylindrical sheaths, the diameter of which is from about 1.2 to about 4 times the maximum diameter of the circle containing said heating means.

12. The arrangement of Claim 10 or 11, wherein each sheet is separated from the next one by a wall of refractory material substantially perpendicular to the axis of the reactor, said walls being arranged so that they define spaces or passages for circulation of the gas mixtures and/or effluent, between them and the sheets formed by the sheaths surrounding the heating means.

13. The arrangement of any of Claims 10-12, wherein the passages between the sheaths and/or between the sheets and/or between the sheets and the walls have a dimension of from about 1 to 100 mm.

14. The arrangement of any of Claims 10-13, wherein the passages are at least partly filled with a lining of ceramic material.

## Patentansprüche

1. Verfahren zum thermischen Umwandeln des Methans in Kohlenwasserstoffe höheren Molekulargewichts in einer Reaktionszone von gemäß einer Richtung länglicher Gestalt, umfassend eine Heizzone und eine Kühlzone, die sich an diese Heizzone anschließt, bei dem man in der Heizzone ein gasförmiges Methan enthaltendes Gemisch gemäß einer Strömungsrichtung zirkulieren läßt, die im wesentlichen parallel zur Richtung der Reaktionszone ist, wobei diese Heizzone eine vielzahl von elektrischen Heizmitteln, die in untereinander im wesentlichen parallelen Lagen angeordnet sind, umfaßt, welche in der Querprojektion ein Bündel mit Dreieck-, Quadrat- oder Rechteckteilung bilden, wobei diese Heizmittel in aufeinanderfolgenden Transversalabschnitten im wesentlichen senkrecht zur Richtung der Reaktionszone gruppiert sind, voneinander unabhängig und mit elektrischer Energie derart gespeist sind, daß wenigstens zwei Teile in der Heizzone bestimmt werden, wobei der erste Teil es ermöglicht, die Charge bis auf eine Temperatur, die höchstens gleich etwa 1500°C ist, zu bringen, und der zweite an den ersten anschließende Teil es ermöglicht, die Charge auf einer Temperatur zu halten, die im wesentlichen gleich der Maximaltemperatur ist, auf die sie in diesem ersten Teil gebracht worden ist und bei dem man die Abströme aus der Heizzone durch Einführen eines Kühlfluids in die Kühlzone kühlt, dann diese Kohlenwasserstoffe höheren Molekulargewichts am Ende der Reaktionszone sammelt, wobei dieses Verfahren sich dadurch auszeichnet, daß die elektrischen Heizmittel gegen den direkten Kontakt mit dem Methan enthaltenden gasförmigen Gemisch durch Hüllen bzw. Hüllrohre isoliert sind, in die man ein Wasserstoff enthaltendes Gas einführt, wobei diese Hüllrohre eine geeignete Permeabilität haben und das Gas in das Innere dieser Hüllen bei einem Druck eingeführt wird, derart, daß sich eine Diffusion wenigstens an gewissen Stellen wenigstens eines Teils des Wasserstoffs vom Inneren dieser Hüllrohre aus gegen das Äußere dieser Hüllrohre einstellt.

2. Verfahren nach Anspruch 1, bei dem man einen ersten Teil der Heizzone bis auf eine Maximaltemperatur von höchstens gleich etwa 1500°C derart erwärmt, daß ein Wärmegradient von etwa 0,5°C/cm bis etwa 25°C/cm über eine Länge von etwa 20 bis 80 % der Gesamtlänge der Heilzone realisiert wird und bei dem man den zweiten an den ersten Teil anschließenden Teil derart erwärmt, daß die Temperaturveränderung längs des gesamten zweiten Teils der Heizzone kleiner als etwa 50°C und vorzugsweise kleiner als etwa 20°C ist.

3. Verfahren nach Anspruch 1 oder 2, bei dem der Druck des Gases in den Hüllrohren um wenigstens 0,1 % größer als der Druck außerhalb dieser Hüllrohre ist.

4. Vorfahren nach einem der Ansprüche 1 bis 3, bei dem die die elektrischen Heizmittel gegen direkten Kontakt mit dem gasförmigen Methan enthaltenden Gemisch isolierenden Hüllrohre aus einem porösen Material gebildet werden, dessen Porosität ausreichend ist, um die Diffusion des Wasserstoffs durch diese Hüllrohre zu erlauben.

5. Verfahren nach Anspruch 5, bei dem die Hüllrohre aus keramischem porösem Material bestehen, das eine offene Porosität von wenigstens etwa 1 Volumenprozent und höchstens etwa 40 Volumenprozent aufweist.

6. Verfahren nach einem der Ansprüche 1 bis 5, bei dem die Heizmittel vom direkten Kontakt mit dem gasförmigen Methan umfassenden Gemisch durch zylindrische Hüllrohre isoliert sind, deren Durchmesser etwa das 1,2- bis etwa 4-Fache des Maximaldurchmessers des Kreises ist, der diese Heizmittel umgibt.

7. Verfahren nach einem der Ansprüche 1 bis 6, bei dem die Abmessung der Durchlässe, in denen das Methan umfassende Gemisch zirkulieren, etwa 1 bis etwa 100 mm ist.

8. Verfahren nach einem der Ansprüche 1 bis 7, bei dem jede Lage von der folgenden durch eine Wandung aus feuerfestem Material getrennt ist.

9. Verfahren nach einem der Ansprüche 1 bis 7, bei dem die Mittel zur elektrischen Beheizung Widerstände aus Molybdänbisilicid aufweisen.

10. Vorrichtung zur Durchführung des Verfahrens nach einem der Ansprüche 1 bis 9, einen Reaktor (1) von gemäß einer Achse länglicher Gestalt, vorzugsweise von quadratischem oder rechteckigem Querschnitt umfassend, der an einem ersten Ende Mittel zum speisen mit gasförmigem Gemisch, am gegenüberliegenden Ende Mittel zum Abzug der erzeugten Abströme und zwischen diesen beiden Enden Mittel zum speisen mit Kühlfluid aufweist, wobei dieser Reaktor in einem ersten Teil (auf der Seite des ersten Endes) eine Vielzahl von von Hüllrohren (4) umgebenen Mitteln zur elektrischen Beheizung (3) aufweist, wobei diese im wesentlichen zueinander parallelen Mittel in im wesentlichen parallelen Lagen senkrecht zur Achse des Reaktors derart angeordnet sind, daß zwischen diesen Hüllrohren und/oder den durch diese Hüllrohre gebildeten Lagen Räume oder Durchlässe für die Zirkulation dar gasförmigen Gemische und/oder der Abströme definiert werden, wobei die Heizmittel und diese Hüllrohre so ausgelegt sind, daß sie diese Durchlässe durch aufeinanderfolgende Querschnitte, die unabhängig und im wesentlichen senkrecht zur Achse des Reaktors sind, erwärmen, wobei dieser Reaktor im übrigen Steuer- und Modulationsmittel für die Heizung umfaßt, die mit diesen Heizmitteln verbunden sind und in einem zweiten Teil (8) (auf der Seite des gegenüberliegenden Endes), angrenzend an diesen ersten Teil, Kühlmittel (9) der Abströme umfassen, die mit diesen Mitteln zur Speisung mit Kühlfluid verbunden sind, wobei die Vorrichtung sich dadurch auszeichnet, daß sie Einführungsmittel, bei einem geeigneten Druck eines Wasserstoff enthaltenden Gases in das Innere der Hüllrohre (4) umfaßt und daß diese Hüllrohre eine ausreichende Permeabilität haben, um wenigstens an gewissen Stellen die Diffusion wenigstens eines Teils des Wasserstoffs vom Inneren dieser Hüllrohre gegen das Äußere dieser Hüllrohre zu ermöglichen.

11. Vorrichtung nach Anspruch 10, bei dem die Mittel zur elektrischen Beheizung umgeben sind von zylindrischen Hüllrohren, deren Durchmesser etwa das 1,2- bis etwa 4-Fache des Maximaldurchmessers des diese Heizmittel umschließenden Kreises ist.

12. Vorrichtung nach einem der Ansprüche 10 oder 11, bei der jede Lage von der folgenden durch eine Wandung aus feuerfestem Material, im wesentlichen senkrecht zur Achse des Reaktors getrennt ist, wobei diese Wandungen derart angeordnet sind, daß zwischen ihnen und den Lagen, die durch die die Heizmittel umschließenden Hüllrohre gebildet sind, Räume oder Durchlässe zur Zirkulation der gasförmigen Gemische und/oder der Abströme gebildet werden.

13. Vorrichtung nach einem der Ansprüche 10 bis 12, bei der die Durchlässe zwischen den Hüllrohren und/oder den Lagen und/oder zwischen den Lagen und den Wandungen eine Abmessung von etwa 1 bis etwa 100 mm haben.

14. Vorrichtung nach einem der Ansprüche 10 bis 13, bei der die Durchlässe wenigstens teilweise mit Auskleidung aus keramischem Material gefüllt sind.
